# EUROPEAN PATENT APPLICATION

(11) **EP 3 444 360 A1**
(43) Date of publication of application: **20.02.2019**
(21) Application number: 18196903.1
(22) Date of filing: 08.08.2012
(51) Int. Cl.: C12Q 1/6879, C12Q 1/6883

(54) **A METHOD OF ANALYSING A BLOOD SAMPLE OF A SUBJECT FOR THE PRESENCE OF A FOETAL DISEASE OR CONDITION MARKER**

(30) Priority: 09.08.2011 US 201161521398 P
(62) Divisional of application: 12748272.7
(71) Applicant: Vereniging Voor Christelijk Hoger Onderwijs, Wetenschappelijk Onderzoek En Patiëntenzorg, 1081 HV Amsterdam (NL)
(72) Inventor: Würdinger, Thomas, 1081 HV Amsterdam (NL); Nilsson, Rolf Jonas, 1081 HV Amsterdam (NL)
(74) Representative: V.O.

(57) **Abstract**

The present invention relates to a method of analysing a blood sample of a subject carrying a foetus for the presence of a foetal disease or condition marker, said method comprising the steps of a) extracting nucleic acid from anucleated blood cells, preferably thrombocytes, in said blood sample to provide an anucleated blood cell-extracted nucleic acid fraction, and b) analysing said anucleated blood cell-extracted nucleic acid fraction for the presence of a foetal disease or condition marker, wherein said foetal disease or condition marker is a nucleic acid of a foetus, or wherein said foetal disease or condition marker is a foetal disease or condition-specific expression profile of genes of a cell of said foetus.

## Description

### FIELD OF THE INVENTION

The invention is in the field of medical diagnostics, in particular in the field of foetal disease or condition diagnostics and monitoring. The invention is directed to methods for detecting foetal disease or condition or condition, and to a method for determining the efficacy of treatment of foetal disease or conditions.

### BACKGROUND OF THE INVENTION

In prenatal screening and diagnostics there is a strong need to be able to predict and detect foetal disease or condition or condition at an early stage. Prenatal tests can identify characteristics of the baby, including size, sex, age, and placement in the uterus, the chance that a baby has certain congenital, genetic, or chromosomal problems, as well as certain types of foetal abnormalities, including some heart problems. Whereas typical prenatal screening tests only reveal the possibility of a problem, there are also diagnostic tests available that can determine whether a foetus has a specific problem. Although more abnormalities can be diagnosed in a foetus than can be treated or cured, it is of great interest to parents who have a family history of genetic disease, to know if their child is healthy or to determine the risk to the child. Also, detecting a foetal disease or condition before delivery may also help in planning the care of the newborn child. If a severe foetal disease or condition or defect or an increased risk of a severe disease, condition or defect is found, the family will have an opportunity to decide on possible termination of pregnancy.

Prior art detection methods for foetal disease or conditions are not without hazard to the mother and/or the unborn child. Such methods include, for instance, Maternal Blood Screening/Triple Screen/ Quadruple Screen, which test is used to screen for Down syndrome and neural tube defects. The test measures Alpha-fetoprotein (AFP), a protein produced by the foetus, which appears in varying amounts in the mother's blood and the amniotic fluid at different times during pregnancy. A deviant level in the mother's blood may indicate a problem. The test also determines the levels of estriol and human chorionic gonadotropin (HCG) ("triple screen" or "triple marker" test), and inhibin-A ("quadruple screen" or "quadruple marker" test). The greater number of markers increases the accuracy of the screening and better identifies the possibility of a problem. Eventually, the test calculates a woman's individual risk based on the levels of the substances in combination with the mother's age, weight, race, presence of insulin dependent diabetes, etc. It's important to note that these screening tests only determine a risk and do not diagnose a condition.

Percutaneous Umbilical Blood Sampling (PUBS), wherein foetal blood is obtained by guiding a needle into the umbilical cord is a diagnostic test. This particular test aims to detect chromosomal disorders. Risks are associated with this procedure, such as miscarriage or infection, so the risks and benefits should be well evaluated. The test is also usually only available after 18 weeks of gestation. Less invasive tests include Contraction Stress Test wherein the uterus is stimulated with Pitocin to determine the effect of contractions on the foetal heart rate. A drawback is that it can induce labour. A Nonstress Test (NST) is used to determine if the baby is responding normally to a stimulus. It is used mostly in high-risk pregnancies or when a health care provider is uncertain of foetal movement. An NST can be performed at any point in the pregnancy after the 26th to 28th week when foetal heart rate can appropriately respond by accelerating and decelerating.

One of the most widely used tests is the Amniocentesis, which involves the withdrawal of amniotic fluid, and the growing of cells in the withdrawn fluid. This test is most often used to detect Down syndrome and other chromosome abnormalities, structural defects such as spina bifida and anencephaly, inherited metabolic disorders as well as other common birth defects, such as heart disorders and cleft lip and palate. The test results are usually available within 1 to 2 weeks. This test can be very accurate. However, the rate of miscarriage with this procedure is considerable. Furthermore, it also carries a risk of uterine infection, which can also cause miscarriage, leakage of amniotic fluid, and injury to the foetus.

In addition to the above, there is Chorionic Villus Sampling (CVS). The chorionic villi are tiny finger-like units that are part of the placenta. They have the same chromosomes and genetic makeup as the foetus. CVS is an alternative to an amniocentesis and involves the transcervical or transabdominal removal of some of the chorionic villi. The biopsy is tested for chromosomal abnormalities, such as Down syndrome. Its advantage over an amniocentesis is that it can be performed earlier, allowing more time for expectant parents to receive counselling and make decisions. The risks associated with CVS include miscarriage, infection and birth defects, and these risks are higher than with amniocentesis. Hence, risks and benefits of the test must be weighed.

The present invention provides a novel method for detecting the presence of foetal disease or conditions in a subject, which aims to overcome the above disadvantages. Further, the present invention aims to provide methods that do not require biopsies, and allow extensive monitoring of both mother and foetus.

### SUMMARY OF THE INVENTION

The present invention in a first aspect provides a method of analysing a blood sample of a subject carrying a foetus for the presence of a foetal disease or condition marker, said method comprising the steps of a) extracting nucleic acid from anucleated blood cells, preferably thrombocytes, in said blood sample to provide an anucleated blood cell-extracted nucleic acid fraction, and b) analysing said anucleated blood cell-extracted nucleic acid fraction for the presence of a foetal disease or condition marker, wherein said foetal disease or condition marker is a nucleic acid of a foetus, or wherein said foetal disease or condition marker is a foetal disease or condition-specific expression profile of genes of a cell of said foetus. Preferably, said foetal disease or condition marker relates to nucleic acids of nucleated cells of said foetus. Preferably the marker is a nucleic acid of a foetus.

The term "anucleated blood cell" as used herein refers to a cell that lacks a nucleus. The term includes reference to both erythrocyte and thrombocyte. Preferred embodiments of anucleated cells in aspects of this invention are thrombocytes. The term "anucleated blood cell" preferably does not include reference to cells that lack a nucleus as a result of faulty cell division.

The term "nucleated cell" as used herein refers to a cell having a nucleus. The term includes reference to somatic cells, germ cells and stem cells, and may include cells from colon, pancreas, brain, bladder, breast, prostate, lung, breast, ovary, uterus, liver, kidney, spleen, thymus, thyroid, nerve tissue, connective tissue, blood, epithelial tissue, lymph node, bone, muscle and skin tissues. The nucleated cell is preferably a cell from a diseased tissue.

Thus, the present invention is - in a highly preferred embodiment - generally aimed at analysing nucleic acids that have been transferred from cells of a foetus, preferably from those that have a nucleus, into cells that have no nucleus, wherein the cells that have no nucleus are present in the blood of the subject carrying the foetus (usually the mother) and which anucleated cells can be easily isolated from the blood stream of said subject and contain nucleic acid from the foetus.

The term "nucleus" refers to the membrane-enclosed organelle found in eukaryotic cells that contains most of the cell's genetic material organized in the form of chromosomes. The genes within these chromosomes are the cell's nuclear genome. The interior of the nucleus contains a number of subnuclear bodies including the RNA-comprising nucleolus, which is mainly involved in the assembly of RNA-comprising ribosomes. After being produced in the nucleolus, ribosomes are exported to the cytoplasm where they translate mRNA.

An anucleated blood cell-extracted nucleic acid fraction preferably refers to a fraction comprising chromosomal DNA, ribosomal RNA, nucleolus RNA, and/or messenger RNA.

The term "gene" as used herein, and in particular in the phrasing "mutation in a gene of a nucleated cell" is meant to refer to any nucleic acid sequence, both chromosomal and extra-chromosomal, of a nucleated cell, preferably a nuclear nucleic acid sequence, and may include transcribed and non-transcribed sequences as well as ribosomal RNA sequences, most preferably chromosomal sequences that are transcribed into RNA.

In a preferred embodiment of a method of the invention said nucleic acid of a foetus is chromosomal or genomic nucleic acid of said foetus, in particular a nucleic acid carrying a mutation that is indicative of a (predisposition to develop) foetal disease or condition in said foetus.

In another preferred embodiment, said foetal disease or condition marker is an expression profile of genes of said foetus, wherein said expression profile is indicative of a (predisposition to develop) foetal disease or condition in said foetus, i.e. a foetal disease or condition-specific expression profile.

In some preferred embodiments of aspects of the present invention, said expression profile of genes is not an expression profile of genes from an anucleated blood cell of said foetus, and is also not an expression profile of genes from a nucleated cell of said subject.

In a preferred embodiment of a method of the invention said foetal disease or condition-specific expression profile is the expression profile of chromosomal genes in (a cell of) said foetus, the expression of which is altered do to the presence of a foetal disease or condition or disorder. Preferably, the profile is the expression profile of chromosomal genes from a nucleated cell of said foetus the mRNA of which is present in anucleated blood cells of the subject carrying the said foetus.

In another preferred embodiment of a method of the invention the foetal nucleic acid is ribonucleic acid (RNA), more preferably messenger ribonucleic acid (mRNA) or miRNA.

In a preferred embodiment of a method of the invention said nucleic acid is not mitochondrial DNA or mitochondrial RNA. Hence, mitochondrial nucleic acid is preferably not an aspect of the present invention.

In another preferred embodiment of a method of analysing a blood sample according to the invention said step b) of analysing said anucleated blood cell-extracted nucleic acid fraction for the presence of a foetal disease or condition marker comprises the selective amplification of i) at least a part of said nucleic acid of a foetal disease or condition agent by (reverse transcriptase) polymerase chain reaction amplification using at least one foetal disease or condition agent-specific amplification primer or probe, or ii) a plurality of mRNAs (or at least a part of the mRNAs) by reverse transcriptase polymerase chain reaction amplification to determine the expression level of the chromosomal genes encoding said mRNAs to thereby provide an expression profile for said genes and comparing said expression profile to a reference profile.

In a preferred embodiment of aspects of the invention the foetal disease or condition is selected from the foetal disease or conditions listed in Table 1.

Table 1. Foetal diseases or conditions subject of embodiments of the present invention.
- Achondroplasia (abnormality of the skeleton causing dwarfism
- Achromatopsia (inability to see color)
- Adrenal Hypoplasia Congenita (reduction in adrenal gland function)
- Adrenoleukodystrophy (progressive brain damage)
- Aicardi Syndrome (partial or complete absence of a key structure in brain)
- Albinism/Hypopigmentation (no melanin pigment in eyes, skin and hair)
- Alexander Disease (neurodegenerative disease)
- Alpers' Disease (degenerative disease of the central nervous system)
- Alpha-1 Antitrypsin Deficiency (decreased A1AT activity in blood & lungs)
- Alzheimer's (degenerative disease starting with memory loss)
- Amblyopia (poor or indistinct vision)
- Angelman Syndrome (intellectual and developmental delay, seizures)
- Anencephaly (absence of a major portion of the brain, skull, and scalp)
- Aniridia (underdevelopment of the eye's iris)
- Anophthalmia (congenital absence of one or both eyes)
- Arnold-Chiari Malformation
- Ataxia Telangiectasia (immunodeficiency disorder)
- Autism (brain development disorder)
- Bardet-Biedl Syndrome (obesity, pigmentary retinopathy, polydactyly, mental retardation, hypogonadism, and renal failure)
- Barth Syndrome (metabolism distortion, delayed motor skills, stamina deficiency, hypotonia, chronic fatigue, delayed growth)
- Batten Disease (fatal, autosomal recessive neurodegenerative disorder)
- Best's Disease (progressive vision loss)
- Beta-thalassemia (may cause anemia)
- Bipolar Disorder (a category of mood disorders)
- Bloom Syndrome (breaks and rearrangements in the chromosomes)
- Branchio-Oto-Renal (BOR) Syndrome (autosomal disorder of kidneys, ears, and neck)
- Canavan Syndrome (progressive damage to nerve cells in the brain)
- Cancer
- Carnitine Deficiencies (metabolic disorders)
- Cerebral Palsy (physical disability in human development)
- Charcot-Marie-Tooth Disease (loss of muscle tissue and touch sensation)
- Cleft Lip/Cleft Palate (abnormal facial development during gestation)
- Coffin Lowry Syndrome (mental retardation and delayed development)
- Coloboma (hole in one of the structures of the eye)
- Color Blindness
- Congenital Heart Defects
- Congenital Hip Dysplasia (Dislocation)
- Connective Tissue Disorders
- Cooley's Anemia/ Thalassemia (formation of abnormal haemoglobin molecules)
- Corneal Dystrophy (non-inflammatory, bilateral opacity of cornea)
- Cornelia de Lange Syndrome (severe developmental anomalies)
- Craniosynostosis
- Cri Du Chat Syndrome
- Cystic Fibrosis (progressive disability due to multisystem failure)
- Cystinosis (autosomal recessive disorder of the renal tubules)
- Dandy-Walker Syndrome
- Developmental Disabilities
- Diabetes
- Down Syndrome or trisomy 21 (impairment of cognitive ability, physical growth & facial appearance)
- Duane Syndrome (inability of the eye to turn out)
- Duchenne Muscular Dystrophy
- Dwarfism
- Ehlers-Danlos Syndrome (defect in collagen synthesis)
- Epidermolysis Bullosa (extremely fragile skin & recurrent blister formation)
- Familial Dysautonomia (disorder of the autonomic nervous system)
- Familial Mediterranean Fever (inflammatory disorder)
- Fanconi Anemia (short stature, skeletal anomalies, bone marrow failure)
- Fetal Alcohol Syndrome
- Fibrodysplasia Ossificans Progressiva (disease of the connective tissue)
- Fragile X Syndrome (X-linked mental retardation)
- Friedreich's Ataxia
- G6PD (Glucose-6-Phosphate Dehydrogenase) Deficiency Anemia
- Galactosemia (inefficient metabolism of the sugar galactose0
- Gaucher Disease (deficiency of the enzyme glucocerebrosidase)
- Gender (sex)
- Gilbert's Syndrome (high levels of unconjugated bilirubin in bloodstream)
- Glaucoma (diseases of the optic nerve)
- Harlequin-type ichthyosis
- Hemochromatosis (excessive absorption of dietary iron)
- Hemoglobin C Disease (abnormal hemoglobin)
- Hemophilia/Bleeding Disorders (inefficient control over blood clotting or coagulation)
- Hirschsprung's Disease (enlargement of the colon)
- Homocystinuria (disorder of the metabolism of the amino acid methionine)
- Huntington's Disease (abnormal body movements)
- Hurler Syndrome (deficiency of alpha-L iduronidase)
- Hydrocephalus
- Klinefelter Syndrome (small testicles and reduced fertility)
- Krabbe Disease (fatal degenerative disorder of nervous system)
- Leber Congenital Amaurosis (loss of vision)
- Leukodystrophies (progressive degeneration of the white matter of brain)
- Long Q-T Syndrome (heart problem)
- Macular Degeneration (loss of central vision)
- Maple Syrup Urine Disease
- Marfan Syndrome (disorder of the connective tissue)
- Marshall-Smith Syndrome (unusual accelerated skeletal maturation)
- McCune-Albright Syndrome (disorder of bones, hormones & skin pigmentation)
- Menkes Disease (disorder that affects copper levels in the body)
- Metabolic Disorders
- Mitochondrial Disease
- Mucolipidoses
- Mucopolysaccharide Disorders
- Muscular Dystrophy (progressive muscle weakness)
- Neonatal Onset Multisystem Inflammatory Disease (uncontrolled inflammation in multiple parts of the body)
- Neural Tube Defects
- Neurofibromatosis (grow of tumors in nerve cells - Schwann cells)
- Niemann-Pick Disease (disorder affecting lipid metabolism)
- Noonan Syndrome (heart malformation, short stature, learning problems)
- Obesity
- Optic Atrophy (loss of some or most of the fibers of the optic nerve)
- Osteogenesis Imperfecta (no protein - collagen, or the ability to make it)
- Peutz-Jeghers Syndrome (benign hamartomatous polyps in gastrointestinal tract)
- Phenylketonuria (PKU) (deficiency in enzyme phenylalanine hydroxylase)
- Polycystic Kidney Disease (multiple cysts in both kidneys)
- Positional Plagiocephaly
- Prader-Willi Syndrome
- Progeria (accelerated aging)
- Pseudoxanthoma Elasticum (fragmentation and mineralization of elastic fibers in tissues)
- Ptosis (drooping upper eyelid or breasts)
- Rentinitis Pigmentosa
- Rett Syndrome
- Scheie Syndrome (absence or malfunctioning of lysosomal enzymes)
- Schizophrenia (impairments in the perception or expression of reality)
- Severe Combined Immunodeficiency (SCID) (crippling of adaptive immune system)
- Sickle Cell Anemia (abnormal, rigid, sickle shape of red blood cells)
- Skeletal Dysplasias (abnormal bone and cartilage development)
- Smith-Magenis Syndrome (developmental disorder)
- Spherocytosis (production of bi-concave disk shaped red blood cells)
- Spina Bifida (incompletely formed spinal cord)
- Spinal Muscular Atrophy
- Spinocerebellar Ataxia (progressive in-coordination of gait)
- Stargardt Disease (Macular Degeneration) (progressive vision loss)
- Stickler Syndrome (disorders affecting connective tissue, mainly collagen)
- Tay-Sachs Disease (usually affects nervous tissue of the brain)
- Thalassemia (inherited anemia)
- Tourette Syndrome
- Treacher Collins Syndrome (craniofacial deformities)
- Trisomy 13 (Patau syndrome)
- Trisomy 18 (Edward syndrome)
- Trisomy 21 (Down syndrome)
- Tuberous Sclerosis (causes benign tumors in various body parts)
- Turner's Syndrome (only one X chromosome in each cell of a female)
- Urea Cycle Disorder (deficiency of one of the enzymes in the urea cycle causing irreversible brain damage and/or death)
- Usher's Syndrome (deafness and a gradual vision loss)
- Velocardiofacial Syndrome (deletion of a small piece of chromosome 22)
- Ventral Wall Defects
- von Hippel-Lindau Disease (abnormal growth of tumors in body parts)
- von Recklinghausen's Disease
- Werner Syndrome (premature aging)
- Williams Syndrome ("elfin" facial appearance, with a low nasal bridge)
- Wilson disease
- Xeroderma Pigmentosum (deficient ability to repair damage caused by ultraviolet (UV) light)
- XXX Syndrome (an extra X chromosome in each cell of a female)
- XYY Syndrome (an extra Y chromosome in each cell of a male)

Also, foetal disease or condition in some aspects of the invention may involve infectious or other diseases of the foetus that may or may not affect the subject carrying the foetus.

In another aspect, the present invention provides a method of diagnosing foetal disease or condition in a subject carrying a foetus using the method of analysing a blood sample according to the invention. Hence, in another preferred embodiment of a method of the invention, said method of analysing a blood sample according to the invention is part of a method of diagnosing foetal disease or condition in a subject carrying a foetus, wherein the presence of said foetal disease or condition marker in said anucleated blood cell-extracted nucleic acid fraction is indicative of said foetus suffering from said foetal disease or condition.

In another aspect, the present invention provides a method for determining the efficacy of a treatment of foetal disease or condition in a foetus, wherein said foetus is carried by a subject, comprising the steps of:
- analysing a blood sample of a subject for the presence of a foetal disease or condition marker using the method of analysing a blood sample according to the invention at a first time point to thereby provide a first value for the level of said foetal disease or condition marker in said subject;
- analysing a blood sample of said subject for the presence of a foetal disease or condition marker using the method of analysing a blood sample according to the invention at a second time point that is earlier or later, preferably later, than said first time point, to thereby provide a second value for the level of said foetal disease or condition marker in said subject, wherein said subject has been subjected to a foetal disease or condition treatment between said first and second time point, and
- comparing said first and second value to determine the efficacy of said foetal disease or condition treatment in said subject.

Foetal disease or condition treatment may comprise treatment with antiviral agents, antibiotic agents, antifungal agents, or antiparasitic agents.

The foetal disease or condition treatment may also comprise treatment of curable diseases.

The skilled artisan will understand that treatment prior to the first time point and subsequent measurements at a second, later, time point without any foetal disease or condition treatment having occurred between said time points, is included in aspects of the invention for determining the efficacy of a foetal disease or condition treatment.

In another aspect, the present invention provides a method for determining the stage of foetal disease or condition. In order to determine the stage of foetal disease or condition, it is beneficial to correlate foetal disease or condition marker values as determined by methods of this invention to foetal disease or condition stages. A single measurement of the foetal disease or condition marker may than be compared to one or more reference values to obtain an indication of the stage of the foetal disease or condition.

In another aspect, the present invention provides a method for determining the stage of foetal disease or condition in a subject, comprising the steps of:
- analysing a blood sample of a subject for the presence of a foetal disease or condition marker using the method of analysing a blood sample of a subject carrying a foetus for the presence of a foetal disease or condition marker according to the present invention as described above to thereby provide a test value for the level of said foetal disease or condition marker in said subject,
- providing a reference value for the level of said foetal disease or condition marker wherein said reference value is correlated to a particular stage of foetal disease or condition, and
- comparing said test and reference value to determine the stage of foetal disease or condition in said subject.

In another aspect, the present invention provides a kit of parts adapted for performing a method of the invention as described herein above, the kit comprising a packaging material comprising at least one of:
- a container for holding anucleated blood cells, preferably thrombocytes, separated from a blood sample;
- an agent for extracting nucleic acids from said anucleated blood cells;
- an agent for selectively amplifying from said nucleic acids extracted from said anucleated blood cells a foetal disease or condition marker (i.e. a foetal disease or condition agent-specific nucleic acid sequence or a foetal disease or condition-specific gene expression profile of chromosomal genes of said subject as indicated above), by (reverse transcriptase) polymerase chain reaction amplification, and
- a printed or electronic instruction for performing a method of the invention as described herein above,
the kit further comprising:
- a reference for said foetal disease or condition marker, wherein said reference is indicative for the presence or absence of said foetal disease or condition marker in said anucleated blood cells-extracted nucleic acid fraction.

In a preferred embodiment of a kit according to the present invention said reference is a reference value for the level of the foetal disease or condition marker or of nucleic acids comprising said foetal disease or condition-specific nucleic acid sequence in anucleated blood cells in a control subject carrying a healthy foetus or in a control subject carrying a foetus suffering from foetal disease or condition, or wherein said reference is a reference expression profile for said plurality of mRNAs in anucleated blood cells from a control subject carrying a healthy foetus or in a control subject carrying a foetus suffering from foetal disease or condition.

In another preferred embodiment of a kit according to the present invention said agent or instruction is selected from a particle or fluorescent marker-labeled anti-anucleated blood cell antibody (preferably a fluorescent marker-labeled anti-thrombocyte antibody), an instruction for bead-based anucleated blood cells isolation (preferably thrombocyte isolation), an instruction for FACS sorting of anucleated blood cells (preferably of thrombocytes), an instruction for anucleated blood cell (preferably thrombocyte) recovery by centrifugation, or negative selection of non-anucleated blood cell components (preferably non-thrombocyte components).

In yet another aspect, the present invention provides a device for diagnosing foetal disease or condition, the device comprising a support and at least one agent for specifically determining a level and/or activity of a foetal disease or condition marker in a anucleated blood cells sample of the subject carrying the foetus, said agent being attached to said support, and a computer-readable medium having computer-executable instructions for performing a method of the invention as described herein above.

In a preferred embodiment of a device according to the present invention, said at least one agent is an oligonucleotide probe or sequencing primer.

In a preferred embodiment of a device according to the present invention, the device comprises a lateral flow device, a dipstick or a cartridge for performing a nucleic acid hybridization reaction between:
- an anucleated blood cells-extracted nucleic acid and at least one foetal disease or condition-specific amplification primer or oligonucleotide probe, wherein said foetal disease or condition-specific amplification primer or oligonucleotide probe hybridizes specifically to a foetal disease or condition-specific nucleic acid sequence, or
- an anucleated blood cells-extracted nucleic acid and a plurality of gene-specific amplification primers or oligonucleotide probes for providing a foetal disease or condition-specific gene expression profile, wherein said gene-specific amplification primers are for genes of a foetus carried by a subject in which the foetal disease or condition is to be diagnosed.

### DETAILED DESCRIPTION OF THE INVENTION

As used herein, the term "foetal disease or condition" refers to a disease, syndrome or disorder, either congenital, genetic or other, from which the foetus suffers or is likely to suffer.

As used herein the term "foetal disease or condition marker" refers to in particular to nucleic acid sequence that is specific for a foetal disease or condition or that can be used to detect the presence of a nucleic acid of a foetal disease or condition in a sample, or to gene expression profile in foetus or a subject carrying said foetus and which profile that is correlated with a foetus (at risk of) suffering from a foetal disease or condition, and wherein the mRNA of said genes is present in the nucleic acid fraction extracted from anucleated blood cells (preferably thrombocytes) of said subject. The term profile includes reference to a ratio of a mutant gene against the normal gene.

As used herein, the term "stage of foetal disease or condition" refers to a qualitative or quantitative assessment of the level of advancement of a foetal disease or condition. Criteria used to determine the stage of a foetal disease or condition include, but are not limited to, the amount of foetal disease or condition marker in the blood of a subject.

As used herein, "nucleic acid" includes reference to a deoxyribonucleotide or ribonucleotide polymer in either single-or double-stranded form, and unless otherwise limited, encompasses known analogues having the essential nature of natural nucleotides in that they hybridize to single-stranded nucleic acids in a manner similar to naturally occurring nucleotides (e. g., peptide nucleic acids).

The term "RNA" refers to ribonucleic acid, a molecule of RNA encoding for a protein product or non-coding for a protein product (such as miRNAs but not excluding other non-coding RNAs). RNA is transcribed from a DNA template.

By "amplified" is meant the construction of multiple copies of a nucleic acid sequence or multiple copies complementary to the nucleic acid sequence using at least one of the nucleic acid sequences as a template. Amplification systems include the polymerase chain reaction (PCR) system, ligase chain reaction (LCR) system, nucleic acid sequence based amplification (NASBA, Cangene, Mississauga, Ontario), Q-Beta Replicase systems, transcription-based amplification system (TAS), and strand displacement amplification (SDA). See, e. g., Diagnostic Molecular Microbiology. Principles and Applications, D. H. Persing et al., Ed., American Society for Microbiology, Washington, D. C. (1993). The product of amplification is termed an amplicon.

The term "hybrid" refers to a double-stranded nucleic acid molecule, or duplex, formed by hydrogen bonding between complementary nucleotides. The terms "hybridise" or "anneal" refer to the process by which single strands of nucleic acid sequences form double-helical segments through hydrogen bonding between complementary nucleotides.

The term "oligonucleotide" refers to a short sequence of nucleotide monomers (usually 6 to 100 nucleotides) joined by phosphorous linkages (e.g., phosphodiester, alkyl and aryl-phosphate, phosphorothioate), or non-phosphorous linkages (e.g., peptide, sulfamate and others). An oligonucleotide may contain modified nucleotides having modified bases (e.g., 5-methyl cytosine) and modified sugar groups (e.g., 2'-O-methyl ribosyl, 2'-0-methoxyethyl ribosyl, 2'-fluoro ribosyl, 2'-amino ribosyl, and the like). Oligonucleotides may be naturally-occurring or synthetic molecules of double- and single-stranded DNA and double- and single-stranded RNA with circular, branched or linear shapes and optionally including domains capable of forming stable secondary structures (e.g., stem-and-loop and loop-stem-loop structures).

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primers will depend on many factors, including temperature and source of primer. A "pair of bi-directional primers" as used herein refers to one forward and one reverse primer as commonly used in the art of DNA amplification such as in PCR amplification.

The term "probe" refers to a single-stranded oligonucleotide sequence that will recognize and form a hydrogen-bonded duplex with a complementary sequence in a target nucleic acid sequence analyte or its cDNA derivative.

The terms "stringency" or "stringent hybridization conditions" refer to hybridization conditions that affect the stability of hybrids, e.g., temperature, salt concentration, pH, formamide concentration and the like. These conditions are empirically optimised to maximize specific binding and minimize nonspecific binding of primer or probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tm) for the specific sequence at a defined ionic strength and pH. The Tₘ is the temperature (under defined ionic strength and pH) at which 50% of a complementary target sequence hybridises to a perfectly matched probe or primer. Typically, stringent conditions will be those in which the salt concentration is less than about 1.0 M Na⁺ ion, typically about 0.01 to 1.0 M Na⁺ ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37°C and a wash in 2x SSC at 40°C. Exemplary high stringency conditions include hybridization in 50% formamide, 1 M NaCl, 1% SDS at 37°C, and a wash in 0.1x SSC at 60°C. Hybridization procedures are well known in the art and are described in e.g. Ausubel et al, Current Protocols in Molecular Biology, John Wiley & Sons Inc., 1994.

"Subject" as used herein includes, but is not limited to, mammals, including, e.g., a human, a non-human primate, a mouse, a pig, a cow, a goat, a cat, a rabbit, a rat, a guinea pig, a hamster, a degu, a horse, a monkey, a sheep, or other non-human mammal; and non-mammal animals, including, e.g., a non-mammalian vertebrate, such as a bird (e.g., a chicken or duck) or a fish, and an invertebrate. The subject carrying a foetus is preferably a gestating mother.

The term "thrombocyte", as used herein, refers to blood platelets, i.e. the small, irregularly-shaped cell fragments do not have a nucleus containing DNA, and that circulate in the blood of mammals. Thrombocytes are 2-3 µm in diameter, and are derived from fragmentation of precursor megakaryocytes. The average lifespan of a thrombocyte is 5 to 9 days. Thrombocytes are involved and play an essential role in haemostasis, leading to the formation of blood clots.

The term "blood" as used herein refers to whole blood (including plasma and cells) and includes arterial, capillary and venous blood.

Proper foetal disease or condition diagnostics is critically depending on disease profiling and the development of diagnostics. However, obtaining easily accessible high-quality nucleic acids for detecting foetal disease or condition remains a significant developmental hurdle. Blood generally contains 150,000-350,000 thrombocytes (platelets) per microliter, providing a highly available biomarker source for research and clinical use. Moreover, thrombocyte isolation is relatively simple and is a standard procedure in blood bank/haematology labs. Since platelets do not contain a nucleus, their RNA transcripts - needed for functional maintenance - are derived from bone marrow megakaryocytes during thrombocyte origination. It has now been found that thrombocytes may take up RNA during circulation via various transfer mechanisms. Cells of foetuses, including cells of those foetuses that suffer from foetal disease or condition, release an abundant collection of genetic material, some of which originates from diseased cells. During circulation in the blood stream thrombocytes absorb the genetic material secreted by the foetus, serving as an attractive platform for the diagnostics of foetal disease or condition.

The present invention provides a novel and easy-to-use method to isolate circulating foetal disease or condition markers as used herein for genetic analysis. The present inventors propose inter alia the isolation of foetal RNA from circulating thrombocytes in the blood of the carrying subject (e.g. mother), yielding pure RNA and an easy way to extract high quality RNA from low amounts of blood. Thrombocyte RNA isolation and subsequent analysis presents a marked increase in the diagnostic sensitivity of circulating RNA in blood. The same accounts for erythrocytes.

These foetal-derived RNAs presents unique genetic information about foetal disease or condition, which may be used to determine foetal disease or condition type, extent of the disease and possibly the susceptibility of the foetal disease or condition to therapeutic treatment. The thrombocyte RNA in the carrying subject can be analyzed for the presence of specific foetus-derived RNAs.

The present invention describes a method of finding specific transcripts derived from cells of foetal disease or condition origin within anucleated cells extracted from blood in the carrying subject. This approach is robust and easy. This is attributed to the rapid and straightforward extraction procedures and the quality of the extracted RNA. Within the clinical setting, extraction or isolation of anucleated cells such as thrombocytes is already implemented in general biological sample collection and therefore it is foreseen that the implementation into the clinic is relatively easy.

The present invention provides a general method for analysing blood of a subject for the presence of a foetal disease or condition nucleic acid marker and a method of diagnosing foetal disease or condition in a subject using said general method. When reference is herein made to a method of the invention, both embodiments are referred to.

A method of the invention can be performed on any suitable body sample comprising anucleated blood cells in the carrying subject, such as for instance a tissue sample comprising blood, but preferably said sample is whole blood.

A blood sample of a subject can be obtained by any standard method, for instance by venous extraction.

The amount of blood needed is not particularly limited. Depending on the methods employed, the skilled person will be capable of establishing the amount of sample required to perform the various steps of the method of the present invention and obtain sufficient nucleic acid for genetic analysis. Generally, such amounts will comprise a volume ranging from 0.01 µl to 100 ml.

The body sample may be analyzed immediately following collection of the sample. Alternatively, analysis according to the method of the present invention can be performed on a stored body sample or on a stored anucleated blood cells fraction thereof. The body sample for testing or the anucleated blood cells fraction thereof can be preserved using methods and apparatuses known in the art. In a collected anucleated blood cell fraction, the thrombocytes are preferably maintenance in inactivated state (ie. in non-activated state). In that way, the cellular integrity and the foetal disease or condition-derived nucleic acids are best preserved.

In case the fraction of anucleated blood cells is a thrombocyte fraction, this platelet isolated fraction does preferably not include platelet poor plasma or platelet rich plasma (PRP). Further isolation of the platelets is preferred for optimal resolution.

The body sample may suitably be processed otherwise, for instance, it may be purified, or digested, or specific compounds may be extracted therefrom. Depending upon the method of characterizing the nucleic acids present in the anucleated blood cells in said sample, which method preferably involves isolation of RNA and RT-PCR, the anucleated blood cells may be extracted from the sample by methods known to the skilled person and be transferred to any suitable medium for extraction of the nucleic acids therefrom should the analysis method so require. The recipient subject's body sample may be treated to remove abundant nucleic acid degrading enzymes (like RNases, DNases) therefrom, in order to prevent early destruction of the thrombocyte nucleic acids.

Anucleated blood cell extraction from the body sample of the foetus-carrying subject may involve any available method. In transfusion medicine, thrombocytes are often collected by apheresis, a medical technology in which the blood of a donor or patient is passed through an apparatus that separates out one particular constituent and returns the remainder to the circulation. The separation of individual blood components is done with a specialized centrifuge. Plateletpheresis (also called thrombopheresis or thrombocytapheresis) is the apheresis process of collecting thrombocytes. Modern automatic plateletpheresis allows blood donors to give a portion of their thrombocytes, while keeping their red blood cells and at least a portion of blood plasma. Although it is possible to provide the body sample comprising anucleated blood cells as envisioned herein by apheresis, it is often easier to collect whole blood and isolate the anucleated blood cells fraction therefrom by centrifugation. Generally, in such a protocol, the thrombocytes are first separated from the other blood cells by a centrifugation step of about 120 x g for about 20 minutes at room temperature to obtain a platelet rich plasma (PRP) fraction. The thrombocytes are then washed (for instance in PBS-EDTA) to remove plasma proteins and enrich for thrombocytes. Wash steps are generally carried out at 850 - 1000 x g for about 10 min at room temperature. Further enrichments can be carried out to yield more pure thrombocyte fractions.

Platelet isolation generally involves blood sample collection in Vacutainer tubes containing anticoagulant citrate dextrose (e.g. 36 ml citric acid, 5 mmol/l KCl, 90 mmol/l NaCl, 5 mmol/l glucose, 10 mmol/l EDTA pH 6.8). A suitable protocol for platelet isolation is described in Ferretti et al. (J Clin Endocrinol Metab 2002; 87:2180-2184). This method involves a preliminary centrifugation step (1,300 rpm per 10 min) to obtain platelet-rich plasma (PRP). Platelets are then washed three times in an antiaggregation buffer (Tris-HCl 10 mmol/l; NaCl 150 mmol/l; EDTA 1 mmol/1; glucose 5 mmol/l; pH 7.4) and centrifuged as above, to avoid any contamination with plasma proteins and to remove any residual erythrocytes. A final centrifugation at 4,000 rpm for 20 min may then be performed to isolate platelets. The platelet pellet may be washed (e.g. in phosphate buffered saline). For quantitative determination of cancer marker levels, the protein concentration of platelet membranes may be used as internal reference. Such protein concentrations may be determined by the method of Bradford (Anal Biochem 1976; 72:248-254), using serum albumin as standard.

Following the provision of the body sample of the subject, and the extraction therefrom of the anucleated blood cells, the anucleated blood cells of the subject are screened for the presence of foetal disease or condition agent-specific nucleic acids (e.g. in the form of specific sequences or in the form of RNA profiles indicative of foetal disease or condition). If foetal disease or condition markers (e.g disease-specific nucleic acids) are encountered in the anucleated blood cells of the subject, or if foetal disease or condition markers are encountered in the anucleated blood cells of the subject at a higher level than in the thrombocytes in a blood sample of a control subject, which foetal disease or condition markers are considered to originate from a foetus suffering from or at risk of suffering from a foetal disease or condition, said foetus (or said subject as the definition may be) is diagnosed with foetal disease or condition as defined herein. Also, if foetal disease or condition-specific nucleic acid (expression) profiles are encountered in the anucleated blood cells of the subject, which nucleic acid (expression) profiles are profiles (expressed) of genes from a foetus suffering from or at risk of suffering from a foetal disease or condition, said subject is diagnosed with foetal disease or condition as defined herein.

A further step in a method of the invention is the provision of an anucleated blood cells-extracted nucleic acid fraction. Such a nucleic acid fraction is subsequently used for the detection of a foetal disease or condition marker therein. An anucleated blood cells-extracted nucleic acid fraction may be obtained by any nucleic acid (NA) extraction method available. Usually NA extraction is performed by using chaotropic reagents. The first step in isolating total NA from cells or tissue is to break open the cells under denaturing conditions. In 1979, Chirgwin et al. (Biochemistry, 18[24]:5294-9, 1979) devised a method for the efficient isolation of total RNA by homogenization in a 4 M solution of the potent protein denaturant guanidinium thiocyanate with 0.1 M 2-mercaptoethanol to break protein disulfide bonds. RNA was then isolated by ethanol extraction or by ultracentrifugation through cesium chloride. In 1987 Chomczynski and Sacchi (Analytical Biochemistry, 162[1]:156-9, 1987) modified this method to devise a rapid single-step extraction procedure using a mixture of guanidinium thiocyanate and phenolchloroform, a method especially useful for processing large numbers of samples or for isolation of RNA from small quantities of cells or tissue. Any commercial kit can also be used for the extraction of RNA, non-limiting examples thereof include Ambion's RNAqueous™ system, Bio101's RNaid Plus kit, Bioline Ltd.'s RNAce kits, CLONTECH's NucleoSpin® RNA II and NucleoTrap mRNA kits, Invitrogen Corp.'s S.N.A.P. Total RNA Isolation Kit and QIAGEN's RNeasy kits.

The detection of a foetal disease or condition marker in the extracted nucleic acid sample may occur by any genetic analysis technique available that is suitable for the detection of foetal disease or condition specific nucleic acid sequences in nucleic acids that are specific for the foetal disease or condition. Usually, such sequences can be easily detected by selective nucleic acid hybridization, involving the formation of a duplex nucleic acid structure formed by selective hybridization with each other of two single-stranded nucleic acid sequences. Selective hybridization includes reference to hybridization, under stringent hybridization conditions, of a nucleic acid sequence to a specified nucleic acid target sequence to a detectably greater degree (e. g., at least 2-fold over background) than its hybridization to non-target nucleic acid sequences and to the substantial exclusion of non-target nucleic acids. Selectively hybridizing sequences typically have about at least 80% sequence identity, preferably 90% sequence identity, and most preferably 100% sequence identity (i. e., complementary) with each other.

Alternatively, detection of a foetal disease or condition-derived nucleic acid may occur through sequencing technologies such as DNA and RNA sequencing.

When detecting foetal disease or condition specific nucleic acid sequences in the form of RNA, it is preferred that the RNA is transcribed into cDNA prior to the detection of the foetal disease or condition specific nucleic acid sequences therein.

RNA can be reverse transcribed into cDNA using RNA-dependent DNA polymerases such as, for example, reverse transcriptases from viruses, retrotransposons, bacteria, etc. These can have RNase H activity, or reverse transcriptases can be used that are so mutated that the RNase H activity of the reverse transcriptase was restricted or is not present (e.g. MMLV-RT RNase H-). RNA-dependent DNA synthesis (reverse transcription) can also be carried by enzymes that show altered nucleic acid dependency through mutation or modified reaction conditions and thus obtain the function of the RNA-dependent DNA polymerase. Commercial kits are available to reverse transcribe RNA into cDNA.

Once the RNA is reverse transcribed into cDNA, the DNA sequence can be analysed for the presence of foetal disease or condition-specific mutations using for instance selective nucleic acid hybridization as described above. Such techniques are well known in the art and may comprise selective amplification using amplification primers that are specific for the mutation to be detected. Alternatively, general primers can be used to amplify the DNA comprising the suspected mutation and the mutation can than be detected in the amplicon by selective nucleic acid hybridization using probes that are specific for the mutation.

Methods of the invention can in principle be performed by using any nucleic acid amplification method, such as the Polymerase Chain Reaction (PCR; Mullis 1987, U.S. Pat. No. 4,683,195, 4,683,202, en 4,800,159) or by using amplification reactions such as Ligase Chain Reaction (LCR; Barany 1991, Proc. Natl. Acad. Sci. USA 88:189-193; EP Appl. No., 320,308), Self-Sustained Sequence Replication (3SR; Guatelli et al., 1990, Proc. Natl. Acad. Sci. USA 87:1874-1878), Strand Displacement Amplification (SDA; U.S. Pat. Nos. 5,270,184, en 5,455,166), Transcriptional Amplification System (TAS; Kwoh et al., Proc. Natl. Acad. Sci. USA 86:1173-1177), Q-Beta Replicase (Lizardi et al., 1988, Bio/Technology 6:1197), Rolling Circle Amplification (RCA; U.S. Pat. No. 5,871,921), Nucleic Acid Sequence Based Amplification (NASBA), Cleavase Fragment Length Polymorphism (U.S. Pat. No. 5,719,028), Isothermal and Chimeric Primer-initiated Amplification of Nucleic Acid (ICAN), Ramification-extension Amplification Method (RAM; U.S. Pat. Nos. 5,719,028 and 5,942,391) or other suitable methods for amplification of DNA.

In order to amplify DNA with a small number of mismatches to one or more of the amplification primers, an amplification reaction may be performed under conditions of reduced stringency (e.g. a PCR amplification using an annealing temperature of 38°C, or the presence of 3.5 mM MgCl₂). The person skilled in the art will be able to select conditions of suitable stringency.

The primers herein are selected to be "substantially" complementary (i.e. at least 65%, more preferably at least 80% perfectly complementary) to their target regions present on the different strands of each specific sequence to be amplified. It is possible to use primer sequences containing e.g. inositol residues or ambiguous bases or even primers that contain one or more mismatches when compared to the target sequence. In general, sequences that exhibit at least 65%, more preferably at least 80% homology with the target DNA oligonucleotide sequences, are considered suitable for use in a method of the present invention. Sequence mismatches are also not critical when using low stringency hybridization conditions.

The detection of the amplification products can in principle be accomplished by any suitable method known in the art. The detection fragments may be directly stained or labelled with radioactive labels, antibodies, luminescent dyes, fluorescent dyes, or enzyme reagents. Direct DNA stains include for example intercalating dyes such as acridine orange, ethidium bromide, ethidium monoazide or Hoechst dyes.

Alternatively, the DNA fragments may be detected by incorporation of labelled dNTP bases into the synthesized DNA fragments. Detection labels which may be associated with nucleotide bases include e.g. fluorescein, cyanine dye or BrdUrd.

When using a probe-based detection system, a suitable detection procedure for use in the present invention may for example comprise an enzyme immunoassay (EIA) format (Jacobs et al., 1997, J. Clin. Microbiol. 35, 791795). For performing a detection by manner of the EIA procedure, either the forward or the reverse primer used in the amplification reaction may comprise a capturing group, such as a biotin group for immobilization of target DNA PCR amplicons on e.g. a streptavidin coated microtiter plate wells for subsequent EIA detection of target DNA amplicons (see below). The skilled person will understand that other groups for immobilization of target DNA PCR amplicons in an EIA format may be employed.

Probes useful for the detection of the target DNA as disclosed herein preferably bind only to at least a part of the DNA sequence region as amplified by the DNA amplification procedure. Those of skill in the art can prepare suitable probes for detection based on the nucleotide sequence of the target DNA without undue experimentation as set out herein. Also the complementary sequences of the target DNA may suitably be used as detection probes in a method of the invention, provided that such a complementary strand is amplified in the amplification reaction employed.

Suitable detection procedures for use herein may for example comprise immobilization of the amplicons and probing the DNA sequences thereof by e.g. southern blotting. Other formats may comprise an EIA format as described above. To facilitate the detection of binding, the specific amplicon detection probes may comprise a label moiety such as a fluorophore, a chromophore, an enzyme or a radio-label, so as to facilitate monitoring of binding of the probes to the reaction product of the amplification reaction. Such labels are well-known to those skilled in the art and include, for example, fluorescein isothiocyanate (FITC), β-galactosidase, horseradish peroxidase, streptavidin, biotin, digoxigenin, ³⁵S or ¹²⁵I. Other examples will be apparent to those skilled in the art.

Detection may also be performed by a so called reverse line blot (RLB) assay, such as for instance described by Van den Brule et al. (2002, J. Clin. Microbiol. 40, 779787). For this purpose RLB probes are preferably synthesized with a 5'amino group for subsequent immobilization on e.g. carboxylcoated nylon membranes. The advantage of an RLB format is the ease of the system and its speed, thus allowing for high throughput sample processing.

The use of nucleic acid probes for the detection of DNA fragments is well known in the art. Mostly these procedures comprise the hybridization of the target DNA with the probe followed by post-hybridization washings. Specificity is typically the function of post-hybridization washes, the critical factors being the ionic strength and temperature of the final wash solution. For DNA-DNA hybrids, the Tₘ (the thermal melting point, i.e. the temperature under defined ionic strength and pH at which 50% of a complementary target sequence hybridizes to a perfectly matched probe) can be approximated from the equation of Meinkoth and Wahl (Anal. Biochem., 138: 267-284 (1984)): Tₘ = 81.5 °C + 16.6 (log M) + 0.41 (% GC)-0.61 (% form)-500/L; where M is the molarity of monovalent cations, % GC is the percentage of guanosine and cytosine nucleotides in the DNA, % form is the percentage of formamide in the hybridization solution, and L is the length of the hybrid in base pairs. The Tₘ is reduced by about 1 °C for each 1 % of mismatching; thus, the hybridization and/or wash conditions can be adjusted to hybridize to sequences of the desired identity. For example, if sequences with > 90% identity are sought, the Tₘ can be decreased 10 °C. Generally, stringent conditions are selected to be about 5 °C lower than the Tₘ for the specific sequence and its complement at a defined ionic strength and pH. However, severely stringent conditions can utilize a hybridization and/or wash at 1, 2, 3, or 4 °C lower than Tₘ; moderately stringent conditions can utilize a hybridization and/or wash at 6,7,8,9, or 10 °C lower than the Tₘ; low stringency conditions can utilize a hybridization and/or wash at 11, 12, 13, 14, 15, or 20 °C lower than Tₘ. Using the equation, hybridization and wash compositions, and desired Tₘ, those of ordinary skill will understand that variations in the stringency of hybridization and/or wash solutions are inherently described. If the desired degree of mismatching results in a Tₘ of less than 45 °C (aqueous solution) or 32 °C (formamide solution) it is preferred to increase the SSC concentration so that a higher temperature can be used. An extensive guide to the hybridization of nucleic acids is found in Tijssen, Laboratory Techniques in Biochemistry and Molecular Biology-Hybridization with Nucleic Acid Probes, Part I, Chapter 2 "Overview of principles of hybridization and the strategy of nucleic acid probe assays", Elsevier. New York (1993); and Current Protocols in Molecular Biology, Chapter 2, Ausubel, et al., Eds., Greene Publishing and Wiley-Interscience, New York (1995).

Detection probes are preferably selected to be "substantially" complementary to one of the strands of the double stranded DNA amplicons generated by an amplification reaction in a method of the invention. Preferably the probes are substantially complementary to the immobilizable (e.g. biotin labelled) antisense strands of the amplicons generated from the target DNA.

It is allowable for detection probes to contain one or more mismatches to their target sequence. In general, sequences that exhibit at least 65%, more preferably at least 80% homology with the target DNA oligonucleotide sequences are considered suitable for use in a method of the present invention.

The step of analysing the thrombocyte-extracted nucleic acid fraction for the presence of a foetal disease or condition-derived nucleic acid can thus be performed by standard nucleic acid analysis techniques. The step of determining whether there is an alteration in the level of said nucleic acid in said nucleic acid fraction with respect to an unaffected blood sample will involve (semi-) quantitative measurements of the amount of the nucleic acid marker or the amount of mRNA in the thrombocytes. A much preferred protocol for the detection of foetal disease or condition-specific mutations in the nucleic acids isolated from thrombocytes is therefore quantitative reverse-transcription PCR (qRT-PCR) (Freeman et al., BioTechniques 26:112-125 (1999)).

An "unaffected blood sample" as referred to above refers to the level of the foetal disease or condition nucleic acid in thrombocytes of a subject carrying a healthy foetus or from the same subject prior to the onset of the foetal disease or condition. Since anucleated blood cell characteristics and quantities of anucleated blood cell components depend on, amongst other things, species and age, it is preferable that the healthy control anucleated blood cells come from a subject of the same species, age and from the same subpopulation (e.g. smoker/nonsmoker). Alternatively, control data may be taken from databases and literature. It will be appreciated that the control sample may also be taken from the subject carrying a foetus suffering from or at risk of suffering from a foetal disease or condition at a particular time-point, in order to analyze the progression of the disease.

Foetal disease or condition-specific markers may include a wide variety of markers known to be associated with foetal disease or condition.

The invention further provides a kit for diagnosing foetal disease or condition in a subject, the kit comprising a packaging material which comprises at least one agent for specifically determining a level and/or activity of at least one nucleic acid mutant in an anucleated blood cell sample of the subject. As used herein, the term "diagnosing" refers to determining the presence of a foetal disease or condition, classifying a foetal disease or condition, determining a severity of foetal disease or condition (grade or stage), monitoring foetal disease or condition progression, forecasting an outcome of the foetal disease or condition and/or prospects of recovery.

It will be appreciated that the tools necessary for detecting the foetal disease or condition-derived nucleic acid may be provided as a kit, such as an FDA-approved kit, which may contain one or more unit dosage form containing the active ingredient for detection of the foetal disease or condition-derived nucleic acid in thrombocytes by a method of the present invention.

Alternatively, the kit may comprise means for collecting the sample and specific amplification and/or detection primers packaged separately.

The kit may be accompanied by instructions for performing a method of the present invention.

For example, the kit may be comprised in a device such as a dipstick or a cartridge, (optionally comprised in a housing) to which a blood sample or an isolated and/or amplified thrombocyte nucleic acid sample may be applied and which detects a foetal disease or condition-derived nucleic acid in said sample. The device may comprise any agent capable of specifically detecting the foetal disease or condition-derived nucleic acid. For example, the device may comprise one or a combination of immobilized mutation-specific hybridization probes that bind the foetal disease or condition-derived nucleic acid and an indicator for detecting binding. In an embodiment of this invention, supports are provided in the device to which the hybridization probes are removably or fixedly attached.

According to one embodiment, the device may be a lateral flow device comprising inlet means for flowing a blood sample or an isolated and/or amplified thrombocyte nucleic acid sample into contact with the agents capable of detecting the foetal disease or condition-derived nucleic acid. The test device can also include a flow control means for assuring that the test is properly operating. Such flow control means can include control nucleic acids bound to a support which capture detection probes added to the sample as a means of confirming proper flow of sample fluid through the test device. Alternatively, the flow control means can include capture probes in the control region which capture control nucleic acids naturally present in said sample or added thereto as control, again indicating that proper flow is taking place within the device.

In another aspect, the present invention provides the use of device of the present invention for diagnosing foetal disease or condition in a subject using any one of the methods described herein above. Very suitable devices for use in diagnosing foetal disease or condition in a subject using any one of the methods described herein above include Platelet RNA chips such as for instance described in Nagalla & Bray (2010) Blood 115 (1): 2-3 and Gnatenko et al. Blood 115 (1): 7-14.

## Claims

1. A method of analysing a blood sample of a subject carrying a foetus for the presence of a foetal disease or condition marker, said method comprising the steps of
a) extracting nucleic acid from anucleated blood cells, preferably thrombocytes, in said blood sample to provide an anucleated blood cell-extracted nucleic acid fraction, and
b) analysing said anucleated blood cell-extracted nucleic acid fraction for the presence of a foetal disease or condition marker, wherein said foetal disease or condition marker is a foetal disease or condition-specific nucleic acid sequence or mutation in the nucleic acid of said foetus, or wherein said foetal disease or condition marker is a foetal disease or condition-specific expression profile of genes of a cell of said foetus.

2. The method of claim 1, wherein said anucleated blood cells are thrombocytes or erythrocytes, preferably thrombocytes.

3. The method of claim 1 or 2, wherein said foetal disease or condition is selected from the group consisting of congential and genetic disorders.

4. The method of claim 3 wherein said genetic disorder is gene mutation of trisomy or wherein said foetal disease or condition is the foetal gender.

5. The method of any one of the preceding claims, wherein said foetal disease or condition marker is a nucleic acid of a foetus.

6. The method of any one of the preceding claims, wherein said expression profile is based on ribonucleic acid (RNA), preferably mRNA in said anucleated blood cell.

7. The method of any one of the preceding claims, wherein said step b) of analysing said anucleated blood cell-extracted nucleic acid fraction for the presence of a foetal disease or condition marker comprises the selective amplification of
i) at least a part of said nucleic acid by (reverse transcriptase) polymerase chain reaction amplification using at least one foetal disease or condition marker-specific amplification primer or probe, or
ii) a plurality of mRNAs (or at least a part of the mRNAs) by reverse transcriptase polymerase chain reaction amplification to determine the expression level of the chromosomal genes encoding said mRNAs to thereby provide an expression profile for said genes and comparing said expression profile to a reference profile.

8. The method of any one of the preceding claims, wherein said method is part of a method of diagnosing said foetal disease or condition in a subject, and wherein the presence of said foetal disease or condition marker in said anucleated blood cell-extracted nucleic acid fraction is indicative of said foetus suffering from or at risk of suffering from said foetal disease or condition.

9. A method for determining the stage of foetal disease or condition or the efficacy of a foetal disease or condition treatment in a subject, comprising the steps of:
- analysing a blood sample of a subject for the presence of a foetal disease or condition marker using the method according to any one of claims 1-8 at a first time point to thereby provide a first value for the level of said foetal disease or condition marker in said subject,
- analysing a blood sample of said subject for the presence of a foetal disease or condition marker using the method according to any one of claims 1-8 at a second time point to thereby provide a second value for the level of said foetal disease or condition marker in said subject, wherein said subject or said foetus has been subjected to a foetal disease or condition treatment between said first and second time point, and
- comparing said first and second value to determine the efficacy of said foetal disease or condition treatment in said subject.

10. A method for determining the stage of a foetal disease or condition in a subject, comprising the steps of:
- analysing a blood sample of a subject carrying a foetus for the presence of a foetal disease or condition marker using the method according to any one of claims 1-8 to thereby provide a test value for the level of said foetal disease or condition marker in said subject,
- providing a reference value for the level of said foetal disease or condition marker wherein said reference value is correlated to a particular stage of foetal disease or condition, and
- comparing said test and reference value to determine the stage of foetal disease or condition in said subject.

11. A kit of parts adapted for performing the method recited in any one of claims 1-10, the kit comprising a packaging material which comprises at least one of:
- a container for holding anucleated blood cells separated from a blood sample of a subject;
- an agent for extracting nucleic acids from said anucleated blood cells;
- an agent for selectively amplifying from said nucleic acids extracted from said anucleated blood cells a foetal disease or condition agent-specific nucleic acid sequence or a foetal disease or condition-specific gene expression profile of chromosomal genes of said subject, and
- a printed or electronic instruction for performing the method recited in any one of claims 1-10,
the kit further comprising:
- a reference for said foetal disease or condition marker, wherein said reference is indicative for the presence or absence of said foetal disease or condition marker in said anucleated blood cells-extracted nucleic acid fraction.

12. The kit of claim 11, wherein said reference is a reference value for the level of nucleic acids comprising said foetal disease or condition agent-specific nucleic acid sequence in anucleated blood cells in a healthy control subject or in a control subject suffering from said foetal disease or condition, or wherein said reference is a reference expression profile for said plurality of mRNAs in anucleated blood cells from a healthy control subject or from a control subject suffering from said foetal disease or condition.

13. A kit of claim 11 or 12, wherein said agent is selected from a particle or fluorescent marker-labeled anti-anucleated blood cell antibody, or wherein said instruction is selected from an instruction for bead-based anucleated blood cells isolation, an instruction for FACS sorting of anucleated blood cells, an instruction for anucleated blood cell recovery by centrifugation, or negative selection of non-anucleated blood cell components.

14. A device for diagnosing foetal disease or condition in a subject, the device comprising a support and at least one agent for specifically determining a level and/or activity of at least one nucleic acid in an anucleated blood cell sample of the subject attached to said support, and
a computer-readable medium having computer-executable instructions for performing the method recited in any one of claims 1-10, preferably wherein said at least one agent is an oligonucleotide probe or sequencing primer.

15. The device of claim 14, comprising a lateral flow device, a dipstick or a cartridge for performing a nucleic acid hybridization reaction between:
- an anucleated blood cells-extracted nucleic acid and at least one foetal disease or condition agent-specific amplification primer or oligonucleotide probe, wherein said foetal disease or condition agent-specific amplification primer or oligonucleotide probe hybridizes specifically to a foetal disease or condition agent-specific nucleic acid sequence, or
- an anucleated blood cells-extracted nucleic acid and a plurality of gene-specific amplification primers or oligonucleotide probes for providing an disease-specific gene expression profile, wherein said genes are genes of a nucleated cell of the subject in which the foetal disease or condition is to be diagnosed.
